# FASCICULE DE BREVET EUROPEEN

(11) **EP 2 785 680 B1**
(45) Date de publication et mention de la délivrance du brevet: **06.01.2016**
(21) Numéro de dépôt: 12788619.0
(22) Date de dépôt: 18.10.2012
(51) Int. Cl.: C07C 213/02, C07C 213/08, C07C 253/30

(54) **PROCÉDÉ AMÉLIORÉ DE SYNTHÈSE DE BIS[3-(N, N-DIALKYLAMINO)PROPY]ÉTHERS**
VERBESSERTES VERFAHREN ZUR SYNTHESE VON BIS-[3-(N,N-DIALKYLAMINO-)PROPYL-]ETHERN
IMPROVED METHOD FOR SYNTHESISING BIS[3-(N,N-DIALKYLAMINO)PROPYL]ETHERS

(30) Priorité: 30.11.2011 FR 1103655
(43) Date de publication de la demande: 08.10.2014
(73) Titulaire: IFP Énergies nouvelles, 92852 Rueil-Malmaison Cedex (FR)
(72) Inventeur: DELFORT, Bruno, 75005 Paris (FR); LE PENNEC, Dominique, 78910 Orgerus (FR); GRANDJEAN, Julien, 69008 Lyon (FR)
(86) Numéro de dépôt international: PCT/FR2012/000424
(87) Numéro de publication internationale: WO 2013/079815

(56) Documents cités:
- US-A- 4 247 482

## Description

### Domaine de l'invention

L'invention concerne la synthèse de diamines tertiaires éthers.

### Contexte

Les diamines tertiaires éthers appartenant à la famille des bis[3-(N,N-dialkylamino)alkyl]éther peuvent présenter de l'intérêt pour différentes applications.

Parmi celles-ci, les bis[3-(N,N-dialkylamino)éthyl]éthers ont suscité beaucoup d'intérêt notamment la bis[3-(N,N-diméthylamino)éthyl]éther ou la 1,2-bis(N,N-diméthylaminoéthoxy)éthane.

Les diamines tertiaires éthers appartenant à la famille bis[3-(N,N-dialkylamino)propyl]éther ont été par contre beaucoup moins étudiées. Elles peuvent également présenter de l'intérêt pour différentes applications.

Les diamines tertiaires éthers appartenant à la famille des bis[3-(N,N-dialkylamino)propyl]éther peuvent être avantageusement utilisées par exemple dans la désacidification de gaz acides que ce soit le gaz naturel ou des gaz de fumées de combustion. On entend par désacidification de gaz acides la réduction de la teneur de ces gaz en composés acides tels que H2S, CO2, COS, CS2.

Les diamines tertiaires éthers appartenant à la famille des bis[3-(N,N-dialkylamino)propyl]éther peuvent entrer dans des formulations conduisant à des polymères notamment les polyuréthanes.

Les diamines tertiaires éthers appartenant à la famille des bis[3-(N,N-dialkylamino)propyl]éther peuvent être des composés précurseurs de molécules trouvant des applications dans d'autres domaines de la chimie.

Parmi les diamines tertiaires éthers appartenant à la famille des bis[3-(N,N-dialkylamino)propyl]éther, deux molécules peuvent présenter un intérêt particulier.
Il s'agit de la bis[3-(N,N-diméthylamino)propyl]éther : 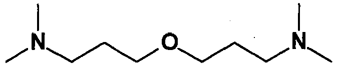 et de la bis[3-(N,N-diéthylamino)propyl]éther :

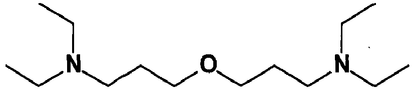

### Art antérieur

L'art antérieur sur la synthèse des bis[3-(N,N-dialkylamino)propyl]éthers est beaucoup moins abondant que celui sur la synthèse des bis[3-(N,N-dialkylamino)éthyl]éthers. La synthèse de ces derniers repose principalement sur la chimie de l'oxyde d'éthylène qui est le précurseur du motif éthoxyéthyle reliant les deux fonctions amines.

Le motif propoxypropyle qui relie les deux fonctions amines des bis[3-(N,N-dialkylamino)éthyl]éthers ne peut pas reposer sur la chimie de l'oxyde de propylène qui conduirait au bis[2-(N,N-dialkylamino)propyl]éthers et non au bis[3-(N,N-dialkylamino)propyl]éthers comme cela est souhaité. En fait, peu de travaux concernant la synthèse des bis[3-(N,N-dialkylamino)propyl]éthers sont disponibles.

La synthèse des bis[3-(N,N-dialkylamino)propyl]éther est décrite par J. Fakstorp et coll. La synthèse de la bis[3-(N,N-diméthylylamino)propyl]éther est décrite dans Acta. Chem. Scand., 11 (1957), 1698-1705 par réaction du 3-(diméthylamino)-propanol avec le 1-chloro-3-(diméthyamino)-propane. Cette réaction résulte de la condensation de l'alcoolate alcalin, par exemple l'alcoolate de sodium, du 3-(diméthylamino)-propanol préparé préalablement sur le 1-chloro-3-(diméthylamino)-propane. En procédant ainsi, pour chaque mole de bis[3-(N,N-diméthylamino)propyl]éther formée, une mole de chlorure alcalin, par exemple de chlorure de sodium sera produite.

Le même auteur décrit dans Acta. Chem. Scand., 8 (1954), 350-353 la synthèse de la bis[3-(N,N-diéthylamino)propyl]éther par réaction du 3-(diéthylamino)-propanol avec le 1-chloro-3-(diéthylamino)-propane dans des conditions similaires.

Le brevet US 3,480,675 (1969) revendique une amélioration de ce mode opératoire en opérant en une seule étape en présence d'une base permettant de générer in situ l'alcoolate du 3-(dialkylamino)-propanol. Ici aussi, une mole de chlorure alcalin, par exemple de chlorure de sodium sera produite par mole de bis[3-(N,N-dialkylamino)propyl]éther.

Ces voies de synthèse utilisent comme réactif le 1-chloro-3-(diméthylamino)-propane ou le 1-chloro-3-(diéthylamino)-propane. Ces molécules résultent de la chloration du 3-(diméthylamino)-propanol ou du 3-(diéthylamino)-propanol. Cette réaction s'effectue au moyen d'un agent de chloration qui peut être par exemple l'acide chlorhydrique, le chlorure de thionyle, le trichlorure de phosphore ou le pentachlorure de phosphore. Ces agents de chloration sont corrosifs et génèrent parfois des sous-produits tel le dioxyde de soufre dans le cas du chlorure de thionyle. Leur utilisation industrielle à grande échelle n'est donc pas souhaitable.

Le brevet US 4,247,482 (1981) propose une voie de synthèse dans laquelle le 3-(dialkylamino)-propanol est d'abord converti en alcoolate de sodium par réaction avec une base, puis converti par réaction avec le trioxyde de soufre en un produit intermédiaire 3-(dialkyamino)-propane-1-sulfate de sodium, lequel par condensation avec l'alcoolate de sodium du 3-(dialkylamino)-propanol conduit au bis[3-(N,N-dialkylamino)propyl]éther en générant une quantité équimolaire de sulfate de sodium.

La formation de sels est inévitable au regard des voies de synthèses décrites par l'art antérieur. Dans tous les cas décrits une mole de sel par mole de bis[3-(N,N-dialkylamino)propyl]éther est fatalement formée. La génération de sels en grande quantité, et qui doivent être éliminés ultérieurement ou dont la gestion est délicate, est devenue incompatible avec le développement de procédés chimiques industriels modernes. Ainsi, les technologies décrites dans l'art antérieur sont, de nos jours, peu compatibles avec la production de bis[3-(N,N-dialkylamino)propyl]éther à grande échelle par voie industrielle.

### Description de l'invention

On a découvert qu'il était possible de préparer des bis[3-(N,N-dialkylamino)propyl]éther selon une procédure ne générant aucun sel et qui est compatible avec un développement industriel.

Cette procédure fait appel à un enchaînement de quatre réactions dont une seule (la dernière) génère un co-produit qui est de l'eau.

L'enchaînement de ces quatre réactions peut s'effectuer en trois ou en quatre étapes.

Le produit de départ utilisé (précurseur) est l'acrylonitrile qui est une molécule industrielle, précurseur de polymères, abondamment disponible dans le monde entier et dont le coût est faible.

### Résumé de l'invention

L'invention concerne un procédé de synthèse de bis[3-(N,N-dialkylamino)propyl]éther à partir d'acrylonitrile comprenant les réactions suivantes:
- première réaction d'addition d'une molécule d'eau et d'une molécule d'acrylonitrile pour produire du 3-hydroxypropionitrile (réaction 1)
- deuxième réaction d'addition d'une molécule de 3-hydroxypropionitrile obtenue par la réaction 1 et d'une molécule d'acrylonitrile pour produire du bis(2-cyanoéthyl)éther (réaction 2);
- réaction d'hydrogénation du bis(2-cyanoéthyl)éther pour effectuer une réduction des fonctions nitriles en fonctions amines primaires) pour produire du bis(3-aminopropyl)éther (réaction 3);
- réaction d'aminoalkylation du bis(3-aminopropyl)éther pour produire du bis[3-(N,N-dialkylamino)propyl]éther (réaction 4).

Dans un mode de réalisation, les première et deuxième réactions d'addition 1 et 2 sont conduites in situ en une seule étape.

Dans un autre mode de réalisation, les première et deuxième réactions d'addition 1 et 2 sont conduites en deux étapes successives.

Dans ce cas, de manière avantageuse, la deuxième réaction d'addition est opérée en présence soit d'un excès de 3-hydroxypropionitrile, soit d'un excès d'acrylonitrile.

De manière avantageuse, la première réaction d'addition est opérée en présence soit d'un excès d'acrylonitrile, soit d'un excès d'eau.

De manière avantageuse, la réaction d'aminoalkylation est réalisée par alkylation réductrice catalytique du bis(3-aminopropyl)éther au moyen d'un aldéhyde en présence d'hydrogène et d'un catalyseur d'hydrogénation.

De préférence, le bis[3-(N,N-dialkylamino)propyl]éther produit est le bis[3-(N,N-diméthylamino)propyl]éther ou le bis[3-(N,N-diéthylamino)propyl]éther.

### Description détaillée de l'invention

### Liste des figures

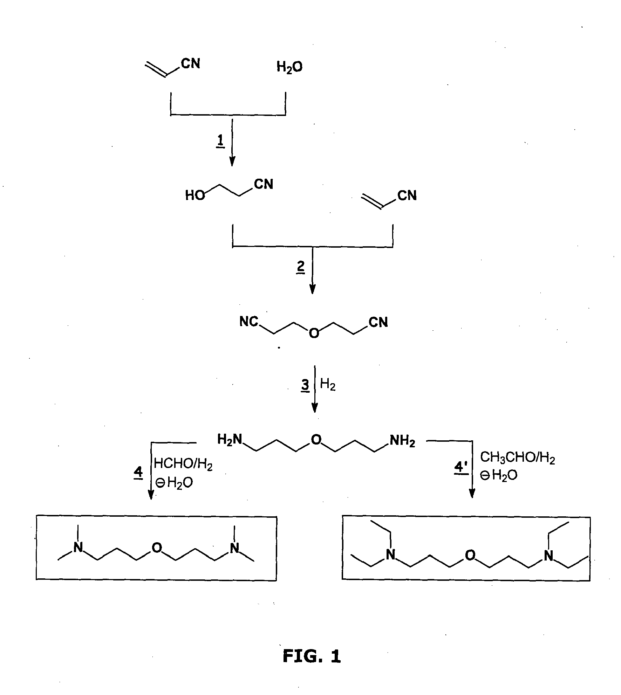

La figure 1 illustre l'invention à titre non limitatif.

La figure 1 représente le procédé de synthèse du bis[3-(N,N-diméthylamino)propyl]éther (voie de gauche) et du bis[3-(N,N-diéthylamino)propyl]éther (voie de droite) à partir de l'acrylonitrile comme produit de départ et comprenant les étapes du procédé selon l'invention.

### Description de la figure 1 :

La figure 1 décrit le procédé de synthèse selon l'invention, à partir d'acrylonitrile comme produit de départ, pour produire du bis[3-(N,N-diméthylamino)propyl]éther (voie de gauche) ou du bis[3-(N,N-diéthylamino)propyl]éther (voie de droite).

### Réactions d'addition (1 et 2)

### Réaction 1 : hydrolyse par addition d'eau et d'acrylonitrile

La première étape consiste en l'addition d'une molécule d'eau et d'une molécule d'acrylonitrile pour conduire au 3-hydroxypropionitrile (réaction 1) selon une réaction connue.

### Réaction 2 : addition du produit de l'étape 1 avec une molécule d'acrylonitrile

Une molécule de 3-hydroxypropionitrile réagit à son tour avec une molécule d'acrylonitrile pour conduire au bis(2-cyanoéthyl)éther (réaction 2).

Les réactions d'addition (1) et (2) peuvent être conduites en deux étapes successives (1) et (2) ou in situ en une seule étape (1+2).
Ces réactions sont des réactions d'additions et ne génèrent aucun sous-produit.
Elles peuvent être favorisées en présence de bases.
Dans le cas où les deux réactions 1 et 2 sont conduites en deux étapes successives, il peut être avantageux dans la réaction 1 d'opérer avec soit un excès d'acrylonitrile soit un excès d'eau. L'un ou l'autre de ces réactifs en excès est aisément séparé, par exemple par distillation à la fin de la réaction 1, puis recyclé dans le processus.
Il peut également être avantageux dans la réaction 2 d'opérer soit avec un excès d'acrylonitrile, soit un excès de 3-hydroxypropionitrile. L'un ou l'autre de ces réactifs en excès est séparé par exemple par distillation à la fin de la réaction 2, puis recyclé dans le processus.

Dans le cas où les réactions 1 et 2 sont conduites in situ en une seule et même étape, il peut être avantageux d'opérer soit avec un excès d'acrylonitrile qui est aisément séparé ultérieurement et recyclé dans le processus, soit avec un excès d'eau qui entraîne la formation additionnelle de 3-hydroxypropionitrile qui est aisément séparé ultérieurement et recyclé dans le processus. L'un ou l'autre de ces réactifs en excès peut être aisément séparé par exemple par distillation à la fin de l'étape.

Les températures d'ébullition de l'acrylonitrile, de l'eau et du 3-hydroxypropionitrile sont respectivement de 77°C, 100°C et 228°C à pression atmosphérique et celle du bis(2-cyanoéthyl)éther est de 110°C sous 66.7 Pa (0.5 mm de mercure) Aussi, les étapes de séparation d'éventuels excès d'un ou de plusieurs des réactifs sont facilement envisageables par distillation. Par conséquent dans ces étapes, le recours éventuel à l'utilisation d'un excès de l'un ou de plusieurs des réactifs pour atteindre la conversion maximale dans des conditions avantageuses de temps et de température est tout à fait compatible avec le processus décrit.

Ces réactions sont décrites notamment dans les brevets US 2,382,036, US 2,448,979 et US 2,816,130 qui décrivent l'addition d'eau et d'acrylonitrile pour conduire au 3-hydroxypropionitrile et au bis(2-cyanoéthyl)éther. Le brevet US 4,965,362 décrit la présence de bases telles des sels d'ammonium pour conduire ces réactions.

Les caractéristiques RMN-13C (CDCl3) du 3-hydroxypropionitrile sont les suivantes :
57.4 ppm : HO-**C**H2-CH2-CN
21.4 ppm : HO-CH2-**C**H2-CN
118.9 ppm : HO-CH2-CH2-**C**N

Les caractéristiques RMN-H (CDCl3) du 3-hydroxypropionitrile sont les suivantes :
3.4 ppm : **H**O-CH2-CH2-CN
3.85 ppm : HO-C**H2**-CH2-CN
2.61 ppm : HO-CH2-C**H2**-CN

Les caractéristiques RMN-13C (CDCl3) du bis(2-cyanoéthyl)éther sont les suivantes :
118.1 ppm : N**C**-CH2-CH2-O-CH2-CH2-**C**N
18.8 ppm : NC-**C**H2-CH2-O-CH2-**C**H2-CN
67.7 ppm : NC-CH2-**C**H2-O-**C**H2-CH2-CN

Les caractéristiques RMN-H (CDCl3) du bis(2-cyanoéthyl)éther sont les suivantes :
2.65 ppm : NC-C**H2**-CH2-O-CH2-C**H2**-CN
3.74 ppm : NC-CH2-C**H2**-O-C**H2**-CH2-CN

### Réaction de réduction des fonctions nitriles par hydrogénation (réaction 3) :

Le bis(2-cyanoéthyl)éther obtenu à l'issue de la réaction 2 est hydrogéné pour conduire au bis(3-aminoproyl)éther selon la réaction bien connue de réduction des fonctions nitriles en fonctions amines primaires (réaction 3). Cette réaction peut s'effectuer avec tous les moyens connus de l'homme du métier dans le respect de la chimie organique.

Elle peut s'effectuer par exemple au moyen d'un hydrure approprié tel l'hydrure mixte de lithium et d'aluminium.

De préférence, la réaction de réduction des fonctions nitriles en fonctions amines primaires est réalisée en présence d'hydrogène, ou en présence d'hydrogène et d'ammoniac au moyen d'un catalyseur approprié tel par exemple des dérivés du nickel.

Cette réaction est une réaction d'addition de l'hydrogène sur la fonction nitrile et ne génère aucun sous produit. De préférence, la réaction est conduite à une température comprise entre 80°C et 190°C et de préférence comprise entre 80°C et 130°C et à une pression comprise entre 5 et 220 bar et de préféence comprise entre 20 et 150 bars.

Le brevet FR 879.788 (1942) décrit l'hydrogénation de l'éther 3,3'-dicyanodiéthylique en présence d'un catalyseur au cobalt, d'ammoniac à une température de 90°C à 100°C pour conduire au bis(3-aminopropyl)éther.
O. F. Wiedman et coll décrivent dans J. Am. Chem. Soc., (1945), p1194 une méthode générale d'hydrogénation du dicyanoéthyléther en diaminopropyléther en présence de nickel de Raney et d'ammoniac entre 50 et 150 bars à une température de 80°C à 125°C.
Cette réaction est également décrite par P. F. Wiley dans J. Am. Chem. Soc., (1946), p1867 à une température de 100°C à 110°C en présence d'ammoniac, de méthanol et de nickel de Raney.
Le brevet US 4,313,004 décrit la réduction de différents dicyanoglycols en présence d'ammoniac et d'un catalyseur à base de nickel entre 90°C et 160°C.
Cette réaction est également décrite dans le brevet Fr 2,367,736 (1977) qui revendique l'hydrogénation du bis(2-cyanoéthyl)éther entre 100°C et 175°C sous une pression comprise entre 34 et 207 bar dans un solvant éther cyclique en présence d'ammoniac et d'un catalyseur à base de nickel ou de cobalt.

Les caractéristiques RMN-13C (CDCl3) du bis(3-aminopropyl)éther sont les suivantes :
39.7 ppm : H2N-**C**H2-CH2-CH2-O-CH2-CH2-**C**H2-NH2
33.8 ppm : H2N-CH2-**C**H2-CH2-O-CH2-**C**H2-CH2-NH2
69.1 ppm : H2N-CH2-CH2-**C**H2-O-**C**H2-CH2-CH2-NH2

Les caractéristiques RMN-H (CDCl3) du bis(3-aminopropyl)éther sont les suivantes :
1.13 ppm : **H2**N-CH2-CH2-CH2-O-CH2-CH2-CH2-N**H2**
2.79 ppm : H2N-C**H2**-CH2-CH2-O-CH2-CH2-C**H2**-NH2
1.17 ppm : H2N-CH2-C**H2**-CH2-O-CH2-C**H2**-CH2-NH2
33.49 ppm : H2N-CH2-CH2-C**H2**-O-C**H2**-CH2-CH2-NH2

### Réaction d'aminoalkylation (réaction 4) :

Enfin, le bis(3-aminopropyl)éther est converti en bis[3-(N,N-diméthylamino)propyl]éther ou en bis[3-(N,N-diéthylamino)propyl]éther selon une réaction d'aminoalkylation qui est dans le cas de la figure 1 une méthylation ou une éthylation (réaction 4 ou 4').

Ces aminoalkylations peuvent être effectuées au moyen de toutes les méthodes permises par la chimie organique et connues de l'homme du métier.

On peut citer par exemple la réaction de méthylation dite d'Eschweiler-Clarke qui requiert un mélange de formaldéhyde et d'acide formique.
On peut également citer la réaction de Leuckart qui utilise une cétone ou un aldéhyde et un formiate d'ammonium.

De manière préférée, l'aminoalkylation est réalisée par la réaction dite d'alkylation réductrice catalytique des amines au moyen d'un aldéhyde, par exemple le formaldéhyde ou l'acétaldéhyde, en présence d'hydrogène et d'un catalyseur approprié choisi parmi les catalyseurs d'hydrogénation tels que par exemple (de manière non limitative) le platine sur charbon, le palladium sur charbon ou des dérivés du nickel, du cuivre ou du chrome tel par exemple le nickel de Raney. Les réactions d'alkylation réductrice catalytique des amines présentent entre autres l'avantage d'être rapides, sélectives et de ne générer comme sous-produit que de l'eau (condensation). Elles sont largement utilisées dans les procédés industriels de fabrication d'alkylamines, telles que les méthylamines ou les éthylamines, et sont compatibles avec les technologies utilisées industriellement.

Plus généralement, la réaction d'aminoalkylation met en oeuvre un aldéhyde ou une cétone, et est avantageusement conduite à une température comprise par exemple entre la température ambiante et 200°C et de préférence à une pression comprise entre la pression atmosphérique et 80 bars. Les conditions expérimentales sont très dépendantes de la nature du système catalytique employé.

On peut par exemple réaliser la méthylation du bis(3-aminopropyl)éther en bis[3-(N,N-diméthylamino)propyl]éther dans un réacteur autoclave par réaction avec du formaldéhyde en excès sous une pression d'hydrogène de 20 bar à 120°C en 3 heures en présence d'un catalyseur au palladium sur charbon.
On peut par exemple réaliser l'éthylation du bis(3-aminoproyl)éther en bis[3-(N,N-diéthylamino)propyl]éther en suivant la même procédure, mais en remplaçant le formaldéhyde par de l'acétaldéhyde.

Les caractéristiques RMN-13C (CDCl3) du bis[3-(N,N-diméthylamino)propyl]éther sont les suivantes :
13C-RMN (CDCl3) :
   27,3ppm : (CH3)2N-CH2-C**H2**-CH2-O-CH2-C**H2**-CH2-N(CH3)2
   44,7ppm : (**CH3**)2N-CH2-CH2-CH2-O-CH2-CH2-CH2-N(C**H3**)2
   55,9ppm : (CH3)2N-C**H2**-CH2-CH2-O-CH2-CH2-**CH2**-N(CH3)2
   68,2ppm : (CH3)2N-CH2-CH2-**CH2**-O-**CH2**-CH2-CH2-N(CH3)2

Les caractéristiques RMN-13C (CDCl3) du bis[3-(N,N-diéthylamino)propyl]éther sont les suivantes :
13C-RMN (CDCl3) :
   11,3ppm : (**CH3**-CH2)2N-CH2-CH2-CH2-O-CH2-CH2-CH2-N(CH2-**CH3**)2
   27,1ppm : (CH3-CH2)2N-CH2-**CH2**-CH2-O-CH2-**CH2**-CH2-N(CH2-CH3)2
   46,3ppm : (CH3-**CH2**)2N-CH2-CH2-CH2-O-CH2-CH2-CH2-N(**CH2**-CH3)2
   49,1ppm : (CH3-CH2)2N-**CH2**-CH2-CH2-O-CH2-CH2-**CH2**-N(CH2-CH3)2
   68,5ppm : (CH3-CH2)2N-CH2-CH2-**CH2**-O-**CH2-**CH2-CH2-N(CH2-CH3)2

### Variantes du procédé de synthèse selon l'invention

Une variante du procédé selon l'invention peut faire appel à l'acrylamide au lieu de l'acrylonitrile, comme produit de départ, les étapes du procédé de synthèse selon l'invention étant telles que décrites précédemment : l'étape d'hydrolyse de l'acrylamide conduit alors au propanamide,3,3'-oxybis qui est hydrogéné en bis(3-aminopropyl)éther puis alkylé, par exemple méthylé ou éthylé comme décrit précédemment.

De même, on peut envisager de faire appel au N,N-diméthylacrylamide ou au N,N-diéthylacrylamide au lieu de l'acrylonitrile. L'étape d'hydrolyse conduit alors au N,N-diméthylpropanamide,3,3'-oxybis ou au N,N-diéthylpropanamide,3,3'-oxybis. Enfin, une étape d'hydrogénation des fonctions amides, suivie ou non d'une alkylation, conduit aux diamines de l'invention.

### Application du procédé de synthèse selon l'invention

Le procédé de synthèse selon l'invention peut être utilisé pour produire des diamines tertiaires éthers appartenant à la famille des bis[3-(N,N-dialkylamino)propyl]éther. Celles-ci peuvent être avantageusement utilisées par exemple dans la désacidification de gaz acides que ce soit le gaz naturel ou des gaz de fumées de combustion. On entend par désacidification de gaz acides la réduction de la teneur de ces gaz en composés acides tels que H₂S, CO₂, COS, CS₂.
Parmi les diamines tertiaires éthers appartenant à la famille des bis[3-(N,N-dialkylamino)propyl]éther et produites au moyen du procédé de synthèse selon l'invention, deux molécules peuvent présenter un intérêt particulier pour la désacidification de gaz acides.

Il s'agit de la bis[3-(N,N-diméthylamino)propyl]éther 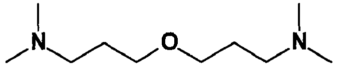 et de la bis[3-(N,N-diéthylamino)propyl]éther

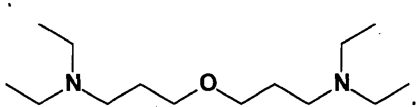

## Revendications

1. Procédé de synthèse de bis[3-(N,N-dialkylamino)propyl]éther à partir d'acrylonitrile comprenant les réactions suivantes:
- première réaction d'addition d'une molécule d'eau et d'une molécule d'acrylonitrile pour produire du 3-hydroxypropionitrile (réaction 1)
- deuxième réaction d'addition d'une molécule de 3-hydroxypropionitrile obtenue par la réaction 1 et d'une molécule d'acrylonitrile pour produire du bis(2-cyanoéthyl)éther (réaction 2);
- réaction d'hydrogénation du bis(2-cyanoéthyl)éther pour effectuer une réduction des fonctions nitriles en fonctions amines primaires) pour produire du bis(3-aminopropyl)éther (réaction 3);
- réaction d'aminoalkylation du bis(3-aminopropyl)éther pour produire du bis[3-(N,N-dialkylamino)propyl]éther (réaction 4).

2. Procédé de synthèse selon la revendication 1 dans lequel les première et deuxième réactions d'addition 1 et 2 sont conduites in situ en une seule étape.

3. Procédé de synthèse selon la revendication 1 dans lequel les première et deuxième réactions d'addition 1 et 2 sont conduites en deux étapes successives.

4. Procédé de synthèse selon la revendication 3 dans lequel la deuxième réaction d'addition est opérée en présence soit d'un excès de 3-hydroxypropionitrile soit d'un excès d'acrylonitrile.

5. Procédé de synthèse selon la revendication 1 à 4 dans lequel la première réaction d'addition est opérée en présence soit d'un excès d'acrylonitrile soit d'un excès d'eau.

6. Procédé selon l'une des revendications précédentes dans lequel la réaction d'aminoalkylation est réalisée par alkylation réductrice catalytique du bis(3-aminopropyl)éther au moyen d'un aldéhyde en présence d'hydrogène et d'un catalyseur d'hydrogénation.

7. Procédé selon l'une des revendications précédentes dans lequel le bis[3-(N,N-dialkylamino)propyl]éther est le bis[3-(N,N-diméthylamino)propyl]éther ou le bis[3-(N,N-diéthylamino)propyl]éther.

## Patentansprüche

1. Verfahren zur Synthese von bis[3-(N,N-Dialkylamino)propyl]ether ausgehend von Acrylnitril umfassend die folgenden Reaktionen:
- erste Additionsreaktion eines Wassermoleküls mit einem Acrylnitrilmolekül, um 3-Hydroxipropionitril herzustellen (Reaktion 1),
- zweite Additionsreaktion eines 3-Hydroxipropionitril-Moleküls erhalten durch Reaktion 1, und eines Acrylnitrilmoleküls, um bis(2-cyanoethyl)ether herzustellen (Reaktion 2),
- Hydrierungsreaktion von bis(2-cyanoethyl)ether, um eine Reduktion der Nitrilfunktionen zu primären Aminfunktionen zu erreichen, um bis(3-aminopropyl)ether zu erhalten (Reaktion 3),
- Aminoalkylierungsreaktion des bis(3-aminopropyl)ether, um bis[3-(N,N-Dialkylamino)propyl]ether herzustellen (Reaktion 4).

2. Syntheseverfahren gemäß Anspruch 1, bei dem die erste und zweite Additionsreaktion in situ in einer einzigen Stufe durchgeführt werden.

3. Syntheseverfahren gemäß Anspruch 1, bei dem die erste und zweite Additionsreaktion in zwei aufeinanderfolgenden Stufen durchgeführt werden.

4. Syntheseverfahren gemäß Anspruch 3, bei dem die zweite Additionsreaktion in Gegenwart von entweder einem Überschuss an 3-Hydroxipropionitril oder einem Überschuss an Acrylnitril durchgeführt wird.

5. Syntheseverfahren gemäß einem der Ansprüche 1 bis 4, bei dem die erste Additionsreaktion in Gegenwart von entweder einem Überschuss an Acrylnitril oder einem Überschuss an Wasser durchgeführt wird.

6. Verfahren gemäß einer der vorhergehenden Ansprüche, bei dem die Aminoalkylierungsreaktion mittels katalytischer reduzierender Alkylierung des bis(3-aminopropyl)ether mit Hilfe eines Aldehyds in Gegenwart von Wasserstoff und einem Hydrierungskatalysator durchgeführt wird.

7. Verfahren gemäß einer der vorhergehenden Ansprüche, bei dem der bis[3-(N,N-Dialkylamino)propyl]ether das bis[3-(N,N-Dimethylamino)propyl]ether oder das bis[3-(N,N-Diethylamino)propyl]ether ist.

## Claims

1. A method for synthesizing bis[3-(N,N-dialkylamino)propyl]ethers from acrylonitrile, comprising the following reactions:
- first addition reaction of a water molecule and an acrylonitrile molecule to produce 3-hydroxypropionitrile (reaction 1),
- second addition reaction of a 3-hydroxypropionitrile molecule obtained by reaction 1 and an acrylonitrile molecule to produce bis(2-cyanoethyl)ether (reaction 2),
- hydrogenation reaction of the bis(2-cyanoethyl)ether to conduct a reduction of the nitrile functions to primary amine functions in order to produce bis(3-aminopropyl)ether (reaction 3),
- aminoalkylation reaction of the bis(3-aminopropyl)ether to produce bis[3-(N,N-dialkylamino)propyl]ether (reaction 4).

2. A synthesis method as claimed in claim 1, wherein first and second addition reactions 1 and 2 are carried out in situ in a single stage.

3. A synthesis method as claimed in claim 1, wherein first and second addition reactions 1 and 2 are carried out in two successive stages.

4. A synthesis method as claimed in claim 3, wherein the second addition reaction is conducted in the presence of either excess 3-hydroxypropionitrile or excess acrylonitrile.

5. A synthesis method as claimed in any one of claims 1 to 4, wherein the first addition reaction is conducted in the presence of either excess acrylonitrile or excess water.

6. A method as claimed in any one of the previous claims, wherein the aminoalkylation reaction is carried out by catalytic reductive alkylation of the bis(3-aminopropyl)ether by means of an aldehyde in the presence of hydrogen and of a hydrogenation catalyst.

7. A method as claimed in any one of the previous claims, wherein the bis[3-(N,N-dialkylamino)propyl]ether produced is bis[3-(N,N-dimethylamino)propyl]ether or bis[3-(N,N-diethylamino)propyl] ether.
